# EUROPEAN PATENT APPLICATION

(11) **EP 4 234 663 A1**
(43) Date of publication of application: **30.08.2023**
(21) Application number: 22158817.1
(22) Date of filing: 25.02.2022
(51) Int. Cl.: C11C 3/00, C11D 1/28, A61K 8/37, C07C 309/62, C11D 1/12, C11D 1/14, A61K 8/34, A61K 8/36, A61K 8/39, A61K 8/60, C11D 1/29

(54) **MACAÚBA OIL FOR THE PRODUCTION OF OLEOCHEMICALS**

(71) Applicant: BASF SE, 67056 Ludwigshafen am Rhein (DE)
(72) Inventor: HUESKEN, Hendrik, 40789 Monheim (DE); SANCHEZ VALDIVIA, Agustin, 40789 Monheim (DE)
(74) Representative: Maiwald GmbH

(57) **Abstract**

The present invention relates to a process of manufacturing fatty acid methyl esters or its sulfonates comprising the step of converting oil extracted from a plant having an oil yield in tons per hectare per year of at least 6 t/ha/yr into fatty acid methyl esters. Further, the present invention relates to fatty acid methyl esters or its sulfonates obtained from the fruits of a plant having an oil yield in tons per hectare per year of at least 6 t/ha/yr and the use thereof in suitable applications.

## Description

The present invention relates to a process of manufacturing fatty acid methyl esters or its sulfonates comprising the step of converting oil extracted from a plant having an oil yield in tons per hectare per year of at least 6 t/ha/yr into fatty acid methyl esters. Further, the present invention relates to fatty acid methyl esters or its sulfonates obtained from the fruits of a plant having an oil yield in tons per hectare per year of at least 6 t/ha/yr and the use thereof in suitable applications.

Numerous oil-based products are derived from renewable materials such as oil palm (principal source of palm oil). While such an approach is advantage since it safes the petroleum deposit it also provides several downsides. One issue is the deforestation in order to plant e.g. oil palm plantations, which aggravates the current climate change. Deforestation further leads to undesired loss of biodiversity and the loss of habitats for local tribes. In addition, particularly oil palms need tropical conditions and preferred temperatures between about 24 to 28 °C, monthly rainfalls of at least 100 mm/m², and a humidity between about 50 to 70%. These factors limit the possibility of a profitable cultivation.

At the same time the demand for renewable oil increases every year since the worldwide consume is increasing. Products derived from renewable oil can be found in every important industrial section, e.g. food products, pharmaceuticals, consumer goods, or energy (biodiesel).

Against this background, there is an ongoing need for a more environmental friendly alternative to known products derived from renewable oil such as palm oil. In particular, it was an object of the present invention to provide a fatty acid methyl ester or its sulfonate having an improved sustainability profile, as well as a process of manufacturing thereof. Further, it was an object of the present invention to provide a fatty acid methyl ester or its sulfonate, wherein the starting material is derived from plants that are less vulnerable against temperature fluctuation, as well as a process of manufacturing thereof. Finally, it was an objection to provide a personal care composition, a cleaning composition, a nutrition formulation, a pharmaceutical formulation, or a crop formulation having an improved sustainability profile, as well as a process of manufacturing thereof. In this connection, a more environmental friendly alternative preferably provides at least one, more preferably at least two, still more preferably at least three, and in particular at least four, of the following impacts: reduced water demand, reduction of the loss of biodiversity, reduction of loss of habitats for local tribes, reduction of deforestation, improved recovery of degraded areas and springs and watersheds, improved retention of moisture in the soil.

It has surprisingly been found that at least one of these objects can be achieved by applying an oil extracted from a plant having an oil yield in tons per hectare per year of at least 6 t/ha/yr.

Thus, according to one aspect, the present invention relates to a process of manufacturing a fatty acid methyl ester composition or a fatty acid methyl ester sulfonate composition, the process comprising the steps
a) converting oil extracted from a plant having an oil yield in tons per hectare per year of at least 6 t/ha/yr into fatty acid methyl esters,
b) optionally converting the fatty acid methyl esters into fatty acid methyl ester sulfonates.

In the following, preferred embodiments of the above process are described in further detail. It is to be understood that each preferred embodiment is relevant on its own as well as in combination with other preferred embodiments.

In a preferred embodiment A1 of the first aspect, the plant is a palm, preferably a palm of the genus *Acrocomia,* more preferably a Macaúba palm, and in particular *Acrocomia aculeata* and/or
the plant is a palm and the oil is extracted from the palm pulp and/or the palm kernel, preferably wherein the plant is Macaúba palm and the oil is extracted from the Macaúba kernel, and in particular wherein the plant is *Acrocomia aculeata* and the oil is extracted from *Acrocomia aculeata* kernel.

In a preferred embodiment A2 of the first aspect, in step a) the conversion is conducted under chemical or enzymatic conditions, preferably under chemical conditions and/or step a) involves a transesterification.

In a preferred embodiment A3 of the first aspect, the fatty acid methyl ester composition provided in step a) comprises at least 40 wt.-%, based on the total weight of the fatty acid methyl ester composition, of C4-C22 fatty acid methyl esters, preferably C6-C20 fatty acid methyl esters, more preferably C8-C18 fatty acid methyl esters, even more preferably C8-C16 fatty acid methyl esters or C16-C18 fatty acid methyl esters, and in particular C10-C16 fatty acid methyl esters
and/or
1 to 20 of wt.-% of a C8 fatty acid methyl ester,
1 to 8 of wt.-% of a C10 fatty acid methyl ester,
30 to 48 wt.-% of a C12 fatty acid methyl ester,
5 to 15 wt.-% of a C14 fatty acid methyl ester,
4 to 13 wt.-% of a C16 fatty acid methyl ester,
15 to 42 wt.-% of a C18 fatty acid methyl ester, and
0 to 5 wt.-% of a C20 fatty acid methyl ester,
each based on the total weight of the fatty acid methyl ester composition.

In a preferred embodiment A4 of the first aspect, the plant has an oil yield in tons per hectare per year in the range of 6 to 30 t/ha/yr, preferably 7 to 20 t/ha/yr, more preferably of 8 to 15 t/ha/yr.

In a preferred embodiment A5 of the first aspect, step b) is conducted and the fatty acid methyl ester sulfonates are selected from the group consisting of linear alkylbenzene sulfonates, alkyl sulfonates, and alpha olefin sulfonates.

In a preferred embodiment A6 of the first aspect, step a) further comprises the step
a.i) blending the oil extracted from a plant having an oil yield in tons per hectare per year of at least 6 t/ha/yr with an oil extracted from a plant having an oil yield in tons per hectare per year of less than 6 t/ha/yr, preferably wherein the oil extracted from a plant having an oil yield in tons per hectare per year of less than 6 t/ha/yr is derived from coconut oil (CNO), palm oil (PO), and/or palm kernel oil (PKO) and/or
wherein the fatty acid methyl ester composition obtained in step a) is blended with a fatty acid methyl ester composition obtained from an oil extracted from a plant having an oil yield in tons per hectare per year of less than 6 t/ha/yr and a subsequent conversion into the respective fatty acid methyl ester composition, preferably wherein the oil extracted from a plant having an oil yield in tons per hectare per year of less than 6 t/ha/yr is derived from coconut oil (CNO), palm oil (PO), and/or palm kernel oil (PKO).

In a preferred embodiment A7 of the first aspect, wherein step b) is conducted and the fatty acid methyl ester sulfonate composition obtained in step b) is blended with a fatty acid methyl ester sulfonate composition obtained from oil extracted from a plant having an oil yield in tons per hectare per year of less than 6 t/ha/yr and a subsequent conversion into the respective fatty acid methyl ester sulfonate composition, preferably wherein the oil extracted from a plant having an oil yield in tons per hectare per year of less than 6 t/ha/yr is derived from coconut oil (CNO), palm oil (PO), and/or palm kernel oil (PKO).

In a second aspect, the present invention relates to a fatty acid methyl ester composition or a fatty acid methyl ester sulfonate composition obtained by a process according to the first aspect.

In a third aspect, the present invention relates to a fatty acid methyl ester composition or a fatty acid methyl ester sulfonate composition obtained from the fruits of a plant having an oil yield in tons per hectare per year of at least 6 t/ha/yr, wherein the oil obtained from the plant is converted into the fatty acid methyl ester or its sulfonate.

In a preferred embodiment C1 of the fourth aspect, the plant is a palm, preferably a palm of the genus *Acrocomia,* more preferably a Macaúba palm, and in particular *Acrocomia aculeata* and/or wherein the oil is obtained by extraction of the fruits, preferably wherein the plant is a palm and the oil is extracted from the palm pulp and/or the palm kernel, more preferably wherein the plant is Macaúba palm and the oil is extracted from the Macaúba kernel, and in particular wherein the plant is *Acrocomia aculeata* and the oil is extracted from *Acrocomia aculeata* kernel.

In a fourth aspect, the present invention relates to the use of oil extracted from fruits of a plant having an oil yield in tons per hectare per year of at least 6 t/ha/yr for manufacturing a fatty acid methyl esters or sulfonates thereof.

In a fifth aspect, the present invention relates to the use of the fatty acid methyl ester composition or the fatty acid methyl ester sulfonate composition according to the second or the third aspect, in a personal care composition, a cleaning composition, a nutrition formulation, a pharmaceutical formulation, or a crops formulation.

In a sixth aspect, the invention relates to a personal care composition, a cleaning composition, a nutrition formulation, a pharmaceutical formulation, or a crop formulation comprising the fatty acid methyl ester composition or the fatty acid methyl ester sulfonate composition according to the second or the third aspect.

### Detailed Description

Before describing in detail exemplary embodiments of the present invention, definitions which are important for understanding the present invention are given.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±10 %, preferably ±8 %, more preferably ±5 %, even more preferably ±2 %. It is to be understood that the term "comprising" and "encompassing" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only. Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below. It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As used herein the term "does not comprise", "does not contain", or "free of" means in the context that the composition of the present invention is free of a specific compound or group of compounds, which may be combined under a collective term, that the composition does not comprise said compound or group of compounds in an amount of more than 0.8 % by weight, based on the total weight of the composition. Furthermore, it is preferred that the composition according to the present invention does not comprise said compounds or group of compounds in an amount of more than 0.5 % by weight, preferably the composition does not comprise said compounds or group of compounds at all.

When referring to compositions and the weight percent of the therein comprised ingredients it is to be understood that according to the present invention the overall amount of ingredients does not exceed 100% (± 1% due to rounding).

The term "personal care composition" refers to any topical and oral product that can be used at least once daily by the costumer as an everyday care product for the human body, e.g. for face, hair, body, or oral care. The personal care composition may comprise one or more active agents, e.g., organic and/or inorganic UV filters, as well as other ingredients or additives, e.g., emulsifiers, emollients, viscosity regulators, stabilizers, preservatives, or fragrances. Suitable daily care composition are according to the present invention, e.g. leave-on face and body care products and rinse-off face and body care products.

Suitable leave-on face and body care products are, e.g. sunscreen compositions, decorative preparations, and skin care preparations.

The term "sunscreen composition" or "sunscreen" refers to any topical product, which absorbs and which may further reflect and scatter certain parts of UV radiation. Thus, the term "sunscreen composition" is to be understood as not only including sunscreen compositions, but also any cosmetic compositions that provide UV protection. The term "topical product" refers to a product that is applied to the skin and can refer, e.g., to sprays, lotions, creams, oils, foams, powders, or gels. According to the present invention the sunscreen composition may comprise one or more active agents, e.g., organic and inorganic UV filters, as well as other ingredients or additives, e.g., emulsifiers, emollients, viscosity regulators, stabilizers, preservatives, or fragrances.

Suitable decorative preparations are, e.g., lipsticks, nail varnishes, eye shadows, mascaras, dry and moist make-up, rouge, powders, depilatory agents and suntan lotions.

Suitable skin care preparations are e.g., moisturizing, refining, and lifting preparations. The cited daily care compositions can be in the form of creams, ointments, pastes, foams, gels, lotions, powders, make-ups, sprays, sticks or aerosols.

The term "UV filter" or "ultraviolet filter" as used herein refers to organic or inorganic compounds, which can absorb and may further reflect and scatter UV radiation caused by sunlight. UV-filter can be classified based on their UV protection curve as UV-A, UV-B, or broadband filters.

In general, UV light can be divided into UV-A radiation (320 - 400 nm) and UV-B radiation (290 - 320 nm). The definition of "broadband" protection (also referred to as broad-spectrum or broad protection) is based on the "critical wavelength". For broadband coverage, UV-B and UV-A protection must be provided. According to the US requirements, a critical wavelength of at least 370 nm is required for achieving broad spectrum protection. The term "critical wavelength" is defined as the wavelength at which the area under the UV protection curve (% protection versus wavelength) represents 90 % of the total area under the curve in the UV region (290-400 nm). For example, a critical wavelength of 370 nm indicates that the protection of the sunscreen composition is not limited to the wavelengths of UV-B, i.e. wavelengths from 290-320 nm, but extends to 370 nm in such a way that 90 % of the total area under the protective curve in the UV region are reached at 370 nm.

Suitable rinse-off face and body care products are, e.g. shampoo, conditioner, shower gel, body scrub, face scrub, and hand soap.

The term "emollient" relates to cosmetic specific oils used for protecting, moisturizing and lubricating the skin. The word emollient is derived from the Latin word *mollire*, to soften. In general, emollients prevent evaporation of water from the skin by forming an occlusive coating. They can be divided into different groups depending on their polarity index.

The term "polarity index" refers to non-polar or polar oils. Non-polar oils are mainly based on hydrocarbons and lack an electronegative element, such as oxygen. In contrast, polar oils contain heteroatoms that differ in electronegativity, which results in a dipole moment. However, such oils are still insoluble in water, i.e. hydrophobic. The polarity index can be determined by measuring the interfacial tension between the respective oil and water.

The term "administration" refers to the application of a sunscreen or daily care composition to the skin of a person.

The prefix Cₙ-Cₘ indicates in each case the possible number of carbon atoms in the group. The term "alkyl" as used herein denotes in each case a linear or branched alkyl group having usually from 1 to 30 carbon atoms, preferably 4 to 26 or of 1 to 6 or of 1 to 3 carbon atoms. Examples of an alkyl group are methyl, ethyl, n-propyl, iso-propyl, n-butyl, 2-butyl, iso-butyl, tert-butyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl.

The term "fatty alcohol" as used herein is directed to linear or branched, preferably linear, primary alcohols. Fatty alcohols may comprise from 4 to 26 carbon atoms. According to the present invention, the term fatty alcohol encompasses saturated and unsaturated alcohol. The double bond of an unsaturated fatty alcohol can give either cis or trans isomers. According to the present invention, the term fatty alcohol encompasses saturated and unsaturated alcohols. 1-Butanol, 1-hexanol, 1-octanol, 1-decanol, lauryl alcohol, myristyl alcohol, cetyl alcohol, palmitoyl alcohol, stearyl alcohol, oleyl alcohol, arachidyl alcohol, behenyl alcohol, erucyl alcohol, lignoceryl alcohol, and ceryl alcohol should be named in this connection.

The term "fatty alcohol-based surfactant" as used herein denotes a surfactant that originates from a reaction of the primary alcohol group of a fatty alcohol.

The term "fatty acid" as used herein is directed to linear or branched, preferably linear, primary carboxylic acids. Fatty acids may comprise from 4 to 26 carbon atoms. According to the present invention, the term fatty acid encompasses saturated and unsaturated acids. The double bond of an unsaturated fatty acid can give either cis or trans isomers. Caprylic acid, capric acid, lauric acid, myristic acid, myristoleic acid, palmitic acid, palmitoleic acid, sapienic acid, stearic acid, oleic acid, elaidic acid, vaccenic acid, linoleic acid, linoelaidic acid, α-Linolenic acid, arachidic acid, arachidonic acid, eicosapentaenoic acid, erucic acid, behenic acid, docosahexaenoic acid, lignoceric acid, and cerotic acid should be named in this connection.

The term "fatty acid-based surfactant" as used herein denotes a surfactant that originates from a reaction of the primary carboxylic group of a fatty acid.

The term "fatty acid methyl ester" as used herein denotes an ester that originates from a reaction of the primary carboxylic group of a fatty acid methanol.

The term "fatty acid methyl ester sulfonate" as used herein denotes an ester that originates from a reaction of the primary carboxylic group of a fatty acid methanol and further comprises at least one sulfonate group.

The term "oil palm" as used herein denotes a species of palm, which is also known as "*E*/*aeis guineensis*". It is the principal source of "palm oil".

The term "coconut tree" as used herein denotes a member of the palm tree family (*Arecaceae*) and is also referred to as *Cocos nucifera*. It is the principal source for "coconut oil".

The term "Macaúba palm" as used herein denotes a species of palm. Exemplary species are known as "*Acrocomia aculeata*" (also known as "macaíba", "boicaiuva", "macaúva", "coco-de-catarro", "coco-baboso", and "coco-de-espinho"), "*Acrocomia hassleri*", and "*Acrocomia totei.* Macaúba palms can grow high, e.g. up to about 15 m. The Macaúba fruit comprises pulp and kernel.

The term "pulp" as used herein refers to inner flesh of a fruit.

The term "kernel" as used herein is interchangeable with "seed" or "almond".

The term "cleaning composition" as used herein encompasses home care formulation, industrial care formulation, and institutional care formulation. Home care formulations are typically used by private costumers, whereas industrial care formulations are typically used by the industry, and institutional care formulations are typically used in e.g. clinics and nursing homes. It is however also possible that the respective formulations can be used in different areas than intended. Hence, the institutional care formulation may also be used by private costumers or the industry and vic verca. Typically cleaning compositions are e.g. for the laundry, dishwashing, hard surface cleaning, food service and kitchen hygiene, food and beverage processing, commercial laundry, sanitation, institutional cleaning, industrial cleaning, and vehicle and transportation care.

The term "nutrition formulation" as used herein encompasses food and feed formulations. The nutrition formulation can have any suitable form, e.g. liquid or solid and can be administered or uptaken in any suitable manner, e.g. orally, parenterally, or rectally.

The term "pharmaceutical formulation" as used herein refers to any suitable pharmaceutical formulation, which may e.g. be administered in any suitable manner such as by oral, transdermal, parenteral, nasal, vaginal, or rectal application. Suitable solid pharmaceutical formulation can be in form of tablets, suppositories, or capsules or in form of a spray. Suitable transdermal pharmaceutical formulations encompass patches or formulations such as sprays, lotions, creams, oils, foams, ointments, powders, or gels. Liquid pharmaceutical formulations are preferably administered orally, parenterally, or nasal.

The term "liquid" as used herein also encompasses semi-solid conditions, wherein the fluid has an increased viscosity (e.g. creamy, gels, ointments).

The term "crop formulation" as used herein encompasses pesticide formulations, fungicide formulations, and herbicide formulations.

The term "oil yield in tons per hectare per year" as used herein is directed to the oil derived from the fruit of the plant via e.g. extraction, wherein the fruit comprises the pulp and the kernel. It refers to the oil produced per hectare. It is to be understood that the value refers to the oil yield obtained from a monoculture, wherein the plants are cultivated under standard conditions, which depend on the respective plant and are known to the skilled person. Hence, in the event that the plant is not cultivated in a monoculture (e.g. on a cattle field), the respective value for this particular cultivation may be reduced. Typically, oil palm has an oil yield in tons per hectare per year of about 3.8 t/ha/yr, rapeseed has an oil yield in tons per hectare per year of about 0.8 t/ha/yr, sunflower has an oil yield in tons per hectare per year of about 0.7 t/ha/yr, and soya has an oil yield in tons per hectare per year of about 0.6 t/ha/yr.

The term "monoculture" as used herein denotes the practice of growing one plant, e.g. Macaúba palm, in a field at a time. On the example of Macaúba palm, about 500 to about 600 palms can be planted per hectare. In this connection, it is preferred that the minimum distance between the tress is about 3.5 to 4.5 meters. This number varies depending on e.g. the soil. The term "agroforestry" as used herein denotes a land use management system in which trees or shrubs are grown around or among other plant such as other trees or other shrubs or crops or pastureland. It is to be understood that not only one further plant can be present in agroforestry. On the example of Macaúba palm, e.g. about 250 to about 360 or about 325 to about 350, trees can be planted per hectare. In this connection, suitable crops that may be planted together with Macaúba palm are exemplarily beans, mandioca, corn, cereals, sunflower, peanut, rapeseed, soya, and mixtures thereof.

The term "silvopastoral" as used herein denotes a land use management system in which trees and optionally forage are planted within the grazing of domesticated animals. On the example of Macaúba palm, e.g. about 275 to about 450 or about 375 to about 400, trees can be planted per hectare.

Preferred embodiments regarding the process of manufacturing a fatty acid methyl ester composition or a fatty acid methyl ester sulfonate composition or a specific fatty acid methyl ester, its sulfonate and blends thereof, as well as the use thereof, and the products comprising the same are described hereinafter. It is to be understood that the preferred embodiments of the invention are preferred alone or in combination with each other.

As indicated above, the present invention relates in one embodiment to a process of manufacturing a fatty acid methyl ester composition or a fatty acid methyl ester sulfonate composition, the process comprising the steps
a) converting oil extracted from a plant having an oil yield in tons per hectare per year of at least 6 t/ha/yr into fatty acid methyl esters,
b) optionally converting the fatty acid methyl esters into fatty acid methyl ester sulfonates.

In a preferred embodiment, the plant is a palm, preferably a palm of the genus *Acrocomia,* more preferably a Macaúba palm, still more preferably *Acrocomia hassleri*, *Acrocomia totei,* and/or *Acrocomia aculeata,* and in particular *Acrocomia aculeata.*

In a preferred embodiment, the plant is a palm and the oil is extracted from the palm pulp and/or the palm kernel.

In a preferred embodiment, the plant is Macaúba palm and the oil is extracted from the Macaúba kernel, preferably wherein the plant is *Acrocomia hassleri*, *Acrocomia totei,* and/or *Acrocomia aculeata* and the oil is extracted from more preferably *Acrocomia hassleri* kernel, *Acrocomia totei*kernel*,* and/or *Acrocomia aculeata* kernel, and in particular wherein the plant is *Acrocomia aculeata* and the oil is extracted from *Acrocomia aculeata* kernel.

In another preferred embodiment, plant is Macaúba palm and the oil is extracted from the Macaúba pulp, and in particular wherein the plant is *Acrocomia aculeata* and the oil is extracted from *Acrocomia aculeata* pulp.

In another preferred embodiment, plant is Macaúba palm and the oil is extracted from the Macaúba pulp and kernel, and in particular wherein the plant is *Acrocomia aculeata* and the oil is extracted from *Acrocomia aculeata* pulp and kernel.

In a preferred embodiment, the plant can sufficiently grow under tropical and subtropical conditions.

In a preferred embodiment, the plant can sufficiently grow in regions from the 30^{th} parallel north to the 28^{th} parallel south, preferably from the 25^{th} parallel north to the 25^{th} parallel south.

In a preferred embodiment, the plant sufficiently grows at a temperature range of 18 to 30 °C, more preferably of 20 to 28 °C. In this connection it is to be understood that the temperature range is the average temperature over one year. Hence, the plant is preferably less vulnerable to temperature fluctuation.

The term "sufficiently grow" as used herein denotes that the claimed oil yield is achievable under standard cultivation.

In addition, particularly oil palm need tropical conditions and preferred temperatures between about 24 to 28 °C, monthly rainfalls of at least 100 mm/m², and a humidity between about 50 to 70%. These factors limit the possibility of a profitable cultivation.

In a preferred embodiment, the process provides a reduced water demand.

In a preferred embodiment, the process provides a reduction of the loss of biodiversity.

In a preferred embodiment, the process provides a reduction of loss of habitats for local tribes.

In a preferred embodiment, the process provides a reduction of deforestation.

In a preferred embodiment, the process provides an improved recovery of degraded areas and/or springs and watersheds.

In a preferred embodiment, the process provides an improved retention of moisture in the soil.

In this connection it is to be understood that the above-outlined reductions or improvements are compared to plants having an oil yield in tons per hectare per year of less than 6 t/ha/yr, preferably compared to oil palm.

In a preferred embodiment, the oil extracted from a plant having an oil yield in tons per hectare per year of at least 6 t/ha/yr is the crude oil, i.e. not further treated after the extraction from the plant.

In another preferred embodiment, the oil extracted from a plant having an oil yield in tons per hectare per year of at least 6 t/ha/yr is the filtered oil, i.e. wherein the crude oil is first filtered by any known in the art filtering systems and then used in the process. A suitable filtration process is e.g. press filtration.

In a preferred embodiment, in step a) the conversion is conducted under chemical or enzymatic conditions, preferably under chemical conditions.

In a preferred embodiment, step a) involves a transesterification. Any suitable transesterification method can be conducted. Preferably, the transesterification is conducted in the presence of methanol providing glycerol and the respective esters. The reaction can further comprise the addition of a catalysts. Preferably, the alcohol is provided in excess.

In a preferred embodiment, the transesterification is preferably performed under enzymatic conditions, preferably at a temperature of 32 to 40 °C.

As a side product of the transesterification glycerol can be provided, preferably after a refine step.

In a preferred embodiment, the process further comprises the step of separating off glycerol.

In a preferred embodiment, the process further comprises a hydrogenation, wherein the double bonds of the fatty acid moieties are completely or partially removed. If the process comprises a complete hydrogenation, the fatty acid composition does not comprise unsaturated fatty acid moieties.

In a preferred embodiment, the fatty acid methyl ester composition provided in step a) comprises at least 40 wt.-%, based on the total weight of the fatty acid methyl ester composition, of C4-C22 fatty acid methyl esters, preferably C6-C20 fatty acid methyl esters, more preferably C8-C18 fatty acid methyl esters, even more preferably C8-C16 fatty acid methyl esters or C16-C18 fatty acid methyl esters, and in particular C10-C16 fatty acid methyl esters.

In a preferred embodiment, the fatty acid methyl ester composition provided in step a) comprises
1 to 20 of wt.-% of a C8 fatty acid methyl ester,
1 to 8 of wt.-% of a C10 fatty acid methyl ester,
30 to 48 wt.-% of a C12 fatty acid methyl ester,
5 to 15 wt.-% of a C14 fatty acid methyl ester,
4 to 13 wt.-% of a C16 fatty acid methyl ester,
15 to 42 wt.-% of a C18 fatty acid methyl ester, and
0 to 5 wt.-% of a C20 fatty acid methyl ester,
each based on the total weight of the fatty acid methyl ester composition. Said fatty acid methyl ester composition is preferably obtained from oil extracted from Macaúba kernel.

In a preferred embodiment, the fatty acid methyl ester composition provided in step a) comprises
3 to 7 wt.-%, preferably 4 to 6 wt.-%, of a C8 fatty acid methyl ester,
2 to 6 wt.-%, preferably 3 to 5 wt.-%, of a C10 fatty acid methyl ester,
36 to 46 wt.-%, preferably 38 to 42 wt.-%, of a C12 fatty acid methyl ester,
6 to 13 wt.-%, preferably 8 to 11 wt.-%, of a C14 fatty acid methyl ester,
5 to 11 wt.-%, preferably 6 to 9 wt.-%, of a C16 fatty acid methyl ester,
25 to 40 wt.-%, preferably 30 to 38 wt.-% of a C18 fatty acid methyl ester, and
0 to 4 wt.-%, preferably 0 to 3 wt.-%, of a C20 fatty acid methyl ester,
each based on the total weight of the fatty acid methyl ester composition. Said fatty acid methyl ester composition is preferably obtained from oil extracted from Macaúba kernel.

In a preferred embodiment, the fatty acid methyl ester composition provided in step a) comprises
0 to 5 wt.-%, preferably 0 to 3 wt.-%, and in particular 0 to 2 wt.-%, of a C10 fatty acid methyl ester,
0 to 6 wt.-%, preferably 0 to 5 wt.-%, and in particular 1 to 4 wt.-%, of a C12 fatty acid methyl ester,
0 to 6 wt.-%, preferably 0 to 5 wt.-%, and in particular 1 to 4 wt.-%, of a C14 fatty acid methyl ester,
10 to 35 wt.-%, preferably 13 to 32 wt.-%, and in particular 15 to 30 wt.-%, of a C16 fatty acid methyl ester,
55 to 85 wt.-%, preferably 60 to 80 wt.-%, and in particular 65 to 75 wt.-%, of a C18 fatty acid methyl ester,
0 to 4 wt.-%, preferably 0 to 3 wt.-%, and in particular 0 to 2 wt.-%, of a C20 fatty acid methyl ester,
each based on the total weight of the fatty acid methyl ester composition. Said fatty acid methyl ester composition is preferably obtained from oil extracted from Macaúba pulp.

In a preferred embodiment, the fatty acid methyl ester composition provided in step a) comprises at least 85 wt.-% based on the total weight of the fatty acid methyl ester composition, of C4-C22 fatty acid methyl esters, preferably C10-C22 fatty acid methyl esters, more preferably C12-C20 fatty acid methyl esters, even more preferably C12-C20 fatty acid methyl esters, and in particular C12-C18 fatty acid methyl esters.

In a preferred embodiment, the fatty acid methyl ester composition provided in step a) comprises at least 10 wt.-% of C16 fatty acid methyl esters and at least 60 wt.-% of C18 fatty acid methyl esters, each based on the total weight of the fatty acid methyl ester composition.

In a preferred embodiment, the fatty acid methyl ester composition provided in step a) comprises 10 to 40 wt.-% of C16 fatty acid methyl esters and 60 to 90 wt.-% of C18 fatty acid methyl esters, each based on the total weight of the fatty acid methyl ester composition.

In a preferred embodiment, the fatty acid methyl ester composition provided in step a) comprises at least 30 wt.-%, preferably at least 35 wt.-%, and in particular at least 40 wt.-%, based on the total weight of the fatty acid methyl ester composition, of C12-14 fatty acid methyl esters.

In a preferred embodiment, the fatty acid methyl ester composition provided in step a) comprises at least 2 wt.-% of C10 fatty acid methyl esters, at least 25 wt.-% of C12 fatty acid methyl esters, at least 5 wt.-% of C14 fatty acid methyl esters, and at least 4 wt.-% of C16 fatty acid methyl esters, each based on the total weight of the fatty acid methyl ester composition.

In a preferred embodiment, the fatty acid methyl ester composition provided in step a) comprises 0 to 5 wt.-% of C8 fatty acid methyl esters, 2 to 6 wt.-% of C10 fatty acid methyl esters, 25 to 45 wt.-% of C12 fatty acid methyl esters, 5 to 20 wt.-% of C14 fatty acid methyl esters, and 4 to 15 wt.-% of C16 fatty acid methyl esters, each based on the total weight of the fatty acid methyl ester composition.

In a preferred embodiment, the plant has an oil yield in tons per hectare per year in the range of at least 7 t/ha/yr, preferably at least 8 t/ha/yr.

In a preferred embodiment, the plant has an oil yield in tons per hectare per year in the range of 6 to 30 t/ha/yr, preferably 7 to 20 t/ha/yr, more preferably of 8 to 15 t/ha/yr or of 8 to 12 t/ha/y r or of 8 to 11 t/ha/yr.

In a preferred embodiment, step b) is conducted and the fatty acid methyl ester sulfonates are selected from the group consisting of linear alkylbenzene sulfonates, alkyl sulfonates, and alpha olefin sulfonates.

Suitable linear alkylbenzene sulfonates can be expressed by the general formula (I)

CH3-(CH2)n-CHR1-(CH2)m-CO-O-CH3 (I),

wherein n is an integer from 0 to 21, m is an integer from 0 to 21, the sum of n and m is from 3 to 21, R1 is phenyl or benzyl, which is substituted with at least one R2, wherein R2 is SO3M, and wherein M is Na, K, or ammonium. In this connection, it is to be understood that SO3 comprises a negative charge and M comprises a positive charge. Linear alkylbenzene sulfonates are accessible via any suitable known in the art method.

Suitable alkyl sulfonates can be expressed by the general formula (II)

CH3-(CH2)i-CHR3-(CH2)j-CO-O-CH3 (II),

wherein i is an integer from 0 to 21, j is an integer from 0 to 21, the sum of i and j is from 3 to 21, R3 is C1-C10-alkyl, which is substituted with at least one R4, wherein R4 is SO3M, and wherein M is Na, K, or ammonium. Linear alkylbenzene sulfonates are accessible via any suitable known in the art method.

Suitable alpha olefin sulfonates can be expressed by the general formula (III)

R-CHSO3H-CO-O-CH3 (III),

wherein R is saturated or unsaturated C3-C23-alkyl, preferably C5-C19-alkyl. Alpha olefin sulfonates are accessible via any suitable known in the art method.

In a preferred embodiment, the fatty acid methyl ester sulfonate is a beta olefin sulfonate, which can be expressed by the general formula (IV)

R-CHSO3H-CH2-CO-O-CH3 (IV),

wherein R is saturated or unsaturated C2-C22-alkyl, preferably C4-C18-alkyl. Beta olefin sulfonates are accessible via any suitable known in the art method.

In a preferred embodiment, step a) further comprises the step
a.i) blending the oil extracted from a plant having an oil yield in tons per hectare per year of at least 6 t/ha/yr, preferably of at least 7 t/ha/yr, more preferably of at least 8 t/ha/yr, with an oil extracted from a plant having an oil yield in tons per hectare per year of less than 6 t/ha/yr, preferably of less than 5 t/ha/yr, more preferably of less than 4.5 t/ha/yr. In a preferred embodiment, the oil extracted from a plant having an oil yield in tons per hectare per year of 6 to 30 t/ha/yr, more preferably 7 to 20 t/ha/yr, even more preferably of 8 to 15 t/ha/yr or of 8 to 12 t/ha/y r or of 8 to 11 t/ha/yr, is blended with oil extracted from a plant having an oil yield in tons per hectare per year of 0.1 to less than 6 t/ha/yr, preferably of 0.3 to 5 t/ha/yr, more preferably of 0.5 to 4.5 t/ha/yr. In a preferred embodiment, the oil extracted from a plant having an oil yield in tons per hectare per year of less than 6 t/ha/yr is derived from coconut oil (CNO), palm oil (PO), and/or palm kernel oil (PKO). In another preferred embodiment, the oil extracted from a plant having an oil yield in tons per hectare per year of less than 6 t/ha/yr is derived from soy oil, sunflower oil, olive oil, and/or rapeseed oil.

In yet another preferred embodiment the oil extracted from a plant having an oil yield in tons per hectare per year of less than 6 t/ha/yr is derived from coconut oil (CNO), palm oil (PO), palm kernel oil (PKO), soy oil, sunflower oil, olive oil, and/or rapeseed oil.

In a preferred embodiment, the oil extracted from a plant having an oil yield in tons per hectare per year of less than 6 t/ha/yr is derived from coconut oil (CNO), and/or palm kernel oil (PKO).

In a preferred embodiment, the oil extracted from a plant having an oil yield in tons per hectare per year of less than 6 t/ha/yr is derived from palm oil (PO) and/or palm kernel oil (PKO).

In a preferred embodiment, step a) further comprises the step
a.i) blending the oil extracted from a plant having an oil yield in tons per hectare per year of at least 6 t/ha/yr, which is derived from Macaúba kernel, with an oil extracted from a plant having an oil yield in tons per hectare per year of less than 6 t/ha/yr, which is derived from palm kernel oil (PKO).

In a preferred embodiment, step a) further comprises the step
a.i) blending the oil extracted from a plant having an oil yield in tons per hectare per year of at least 6 t/ha/yr, which is derived from Macaúba kernel, with an oil extracted from a plant having an oil yield in tons per hectare per year of less than 6 t/ha/yr, which is derived from coconut oil (CNO).

In a preferred embodiment, step a) further comprises the step
a.i) blending the oil extracted from a plant having an oil yield in tons per hectare per year of at least 6 t/ha/yr, which is derived from Macaúba kernel, with an oil extracted from a plant having an oil yield in tons per hectare per year of less than 6 t/ha/yr, which is derived from palm kernel oil (PKO) and coconut oil (CNO).

In a preferred embodiment, step a) further comprises the step
a.i) blending the oil extracted from a plant having an oil yield in tons per hectare per year of at least 6 t/ha/yr, which is derived from Macaúba pulp, with an oil extracted from a plant having an oil yield in tons per hectare per year of less than 6 t/ha/yr, which is derived from palm oil (PO).

In a preferred embodiment, the fatty acid methyl ester composition obtained in step a) is blended with a fatty acid methyl ester composition obtained from an oil extracted from a plant having an oil yield in tons per hectare per year of less than 6 t/ha/yr (preferably of 0.1 to less than 6 t/ha/yr, preferably of 0.3 to 5 t/ha/yr, more preferably of 0.5 to 4.5 t/ha/yr) and a subsequent conversion into the respective fatty acid methyl ester composition. In a preferred embodiment, the oil extracted from a plant having an oil yield in tons per hectare per year of less than 6 t/ha/yr is derived from coconut oil (CNO), palm oil (PO), and/or palm kernel oil (PKO). In another preferred embodiment, the oil extracted from a plant having an oil yield in tons per hectare per year of less than 6 t/ha/yr is derived from soy oil, sunflower oil, olive oil, and/or rapeseed oil.

In yet another preferred embodiment the oil extracted from a plant having an oil yield in tons per hectare per year of less than 6 t/ha/yr is derived from coconut oil (CNO), palm oil (PO), palm kernel oil (PKO), soy oil, sunflower oil, olive oil, and/or rapeseed oil.

In a preferred embodiment, the oil extracted from a plant having an oil yield in tons per hectare per year of less than 6 t/ha/yr is derived from coconut oil (CNO), and/or palm kernel oil (PKO).

In a preferred embodiment, the oil extracted from a plant having an oil yield in tons per hectare per year of less than 6 t/ha/yr is derived from palm oil (PO) and/or palm kernel oil (PKO).

In a preferred embodiment, the fatty acid methyl ester composition obtained in step a), which is obtained from an oil extracted from a plant having an oil yield in tons per hectare per year of at least 6 t/ha/y, which is derived from Macaúba kernel, is blended with a fatty acid methyl ester composition obtained from an oil extracted from a plant having an oil yield in tons per hectare per year of less than 6 t/ha/yr, which is derived from palm kernel oil (PKO).

In a preferred embodiment, the fatty acid methyl ester composition obtained in step a), which is obtained from an oil extracted from a plant having an oil yield in tons per hectare per year of at least 6 t/ha/y, which is derived from Macaúba kernel, is blended with a fatty acid methyl ester composition obtained from an oil extracted from a plant having an oil yield in tons per hectare per year of less than 6 t/ha/yr, which is derived from coconut oil (CNO).

In a preferred embodiment, the fatty acid methyl ester composition obtained in step a), which is obtained from an oil extracted from a plant having an oil yield in tons per hectare per year of at least 6 t/ha/y, which is derived from Macaúba kernel, is blended with a fatty acid methyl ester composition obtained from an oil extracted from a plant having an oil yield in tons per hectare per year of less than 6 t/ha/yr, which is derived from palm kernel oil (PKO) and coconut oil (CNO).

In a preferred embodiment, the fatty acid methyl ester composition obtained in step a), which is obtained from an oil extracted from a plant having an oil yield in tons per hectare per year of at least 6 t/ha/y, which is derived from Macaúba pulp, is blended with a fatty acid methyl ester composition obtained from an oil extracted from a plant having an oil yield in tons per hectare per year of less than 6 t/ha/yr, which is derived from palm oil (PO).

In a preferred embodiment, wherein step b) is conducted, the fatty acid methyl ester sulfonate composition obtained in step b) is blended with a fatty acid methyl ester sulfonate composition obtained from oil extracted from a plant having an oil yield in tons per hectare per year of less than 6 t/ha/yr and a subsequent conversion into the respective fatty acid methyl ester sulfonate composition. In this connection it is to be understood that the subsequent conversion preferably provides a fatty acid methyl ester sulfonate composition, which is equal to the fatty acid methyl ester sulfonate composition obtained in step d) (however obtained from a plant having an oil yield in tons per hectare per year of less than 6 t/ha/yr). Hence, if step b) provides an alpha olefin sulfonate composition, the additional fatty acid methyl ester sulfonate composition obtained from oil extracted from a plant having an oil yield in tons per hectare per year of less than 6 t/ha/yr is preferably also an alpha olefin sulfonate composition.

In a preferred embodiment, the oil extracted from a plant having an oil yield in tons per hectare per year of less than 6 t/ha/yr is derived from coconut oil (CNO), palm oil (PO), and/or palm kernel oil (PKO). In another preferred embodiment, the oil extracted from a plant having an oil yield in tons per hectare per year of less than 6 t/ha/yr is derived from soy oil, sunflower oil, olive oil, and/or rapeseed oil.

In yet another preferred embodiment the oil extracted from a plant having an oil yield in tons per hectare per year of less than 6 t/ha/yr is derived from coconut oil (CNO), palm oil (PO), palm kernel oil (PKO), soy oil, sunflower oil, olive oil, and/or rapeseed oil.

In a preferred embodiment, the oil extracted from a plant having an oil yield in tons per hectare per year of less than 6 t/ha/yr is derived from coconut oil (CNO), and/or palm kernel oil (PKO).

In a preferred embodiment, the oil extracted from a plant having an oil yield in tons per hectare per year of less than 6 t/ha/yr is derived from palm oil (PO) and/or palm kernel oil (PKO).

In a preferred embodiment, the fatty acid methyl ester sulfonate composition obtained in step b), which is obtained from an oil extracted from a plant having an oil yield in tons per hectare per year of at least 6 t/ha/y, which is derived from Macaúba kernel, is blended with a fatty acid methyl ester sulfonate composition obtained from an oil extracted from a plant having an oil yield in tons per hectare per year of less than 6 t/ha/yr, which is derived from palm kernel oil (PKO).

In a preferred embodiment, the fatty acid methyl ester sulfonate composition obtained in step b), which is obtained from an oil extracted from a plant having an oil yield in tons per hectare per year of at least 6 t/ha/y, which is derived from Macaúba kernel, is blended with a fatty acid methyl ester sulfonate composition obtained from an oil extracted from a plant having an oil yield in tons per hectare per year of less than 6 t/ha/yr, which is derived from coconut oil (CNO).

In a preferred embodiment, the fatty acid methyl ester sulfonate composition obtained in step b), which is obtained from an oil extracted from a plant having an oil yield in tons per hectare per year of at least 6 t/ha/y, which is derived from Macaúba kernel, is blended with a fatty acid methyl ester sulfonate composition obtained from an oil extracted from a plant having an oil yield in tons per hectare per year of less than 6 t/ha/yr, which is derived from palm kernel oil (PKO) and coconut oil (CNO).

In a preferred embodiment, the fatty acid methyl ester sulfonate composition obtained in step b), which is obtained from an oil extracted from a plant having an oil yield in tons per hectare per year of at least 6 t/ha/y, which is derived from Macaúba pulp, is blended with a fatty acid methyl ester sulfonate composition obtained from an oil extracted from a plant having an oil yield in tons per hectare per year of less than 6 t/ha/yr, which is derived from palm oil (PO).

As indicated above, the present invention further relates to a fatty acid methyl ester composition or a fatty acid methyl ester sulfonate composition obtained by the above-outlined process.

In a preferred embodiment, the fatty acid methyl ester composition comprises
1 to 20 of wt.-% of a C8 fatty acid methyl ester,
1 to 8 of wt.-% of a C10 fatty acid methyl ester,
30 to 48 wt.-% of a C12 fatty acid methyl ester,
5 to 15 wt.-% of a C14 fatty acid methyl ester,
4 to 13 wt.-% of a C16 fatty acid methyl ester,
15 to 42 wt.-% of a C18 fatty acid methyl ester, and
0 to 5 wt.-% of a C20 fatty acid methyl ester,
each based on the total weight of the fatty acid methyl ester composition. Said fatty acid methyl ester composition is preferably obtained from oil extracted from Macaúba kernel.

In a preferred embodiment, the fatty acid methyl ester composition comprises
3 to 7 wt.-%, preferably 4 to 6 wt.-%, of a C8 fatty acid methyl ester,
2 to 6 wt.-%, preferably 3 to 5 wt.-%, of a C10 fatty acid methyl ester,
36 to 46 wt.-%, preferably 38 to 42 wt.-%, of a C12 fatty acid methyl ester,
6 to 13 wt.-%, preferably 8 to 11 wt.-%, of a C14 fatty acid methyl ester,
5 to 11 wt.-%, preferably 6 to 9 wt.-%, of a C16 fatty acid methyl ester,
25 to 40 wt.-%, preferably 30 to 38 wt.-% of a C18 fatty acid methyl ester, and
0 to 4 wt.-%, preferably 0 to 3 wt.-%, of a C20 fatty acid methyl ester,
each based on the total weight of the fatty acid methyl ester composition. Said fatty acid methyl ester composition is preferably obtained from oil extracted from Macaúba kernel.

In a preferred embodiment, the fatty acid methyl ester composition comprises
0 to 5 wt.-%, preferably 0 to 3 wt.-%, and in particular 0 to 2 wt.-%, of a C10 fatty acid methyl ester,
0 to 6 wt.-%, preferably 0 to 5 wt.-%, and in particular 1 to 4 wt.-%, of a C12 fatty acid methyl ester,
0 to 6 wt.-%, preferably 0 to 5 wt.-%, and in particular 1 to 4 wt.-%, of a C14 fatty acid methyl ester,
10 to 35 wt.-%, preferably 13 to 32 wt.-%, and in particular 15 to 30 wt.-%, of a C16 fatty acid methyl ester,
55 to 85 wt.-%, preferably 60 to 85 wt.-%, and in particular 65 to 75 wt.-%, of a C18 fatty acid methyl ester,
0 to 4 wt.-%, preferably 0 to 3 wt.-%, and in particular 0 to 2 wt.-%, of a C20 fatty acid methyl ester,
each based on the total weight of the fatty acid methyl ester composition. Said fatty acid methyl ester composition is preferably obtained from oil extracted from Macaúba pulp.

In a preferred embodiment, the fatty acid methyl ester composition comprises at least 85 wt.-% based on the total weight of the fatty acid methyl ester composition, of C4-C22 fatty acid methyl esters, preferably C10-C22 fatty acid methyl esters, more preferably C12-C20 fatty acid methyl esters, even more preferably C12-C20 fatty acid methyl esters, and in particular C12-C18 fatty acid methyl esters.

In a preferred embodiment, the fatty acid methyl ester composition comprises at least 10 wt.-% of C16 fatty acid methyl esters and at least 65 wt.-% of C18 fatty acid methyl esters, each based on the total weight of the fatty acid methyl ester composition.

In a preferred embodiment, the fatty acid methyl ester composition comprises 10 to 40 wt.-% of C16 fatty acid methyl esters and 40 to 90 wt.-% of C18 fatty acid methyl esters, each based on the total weight of the fatty acid methyl ester composition.

In a preferred embodiment, the fatty acid methyl ester composition comprises at least 30 wt.-%, preferably at least 35 wt.-%, and in particular at least 40 wt.-%, based on the total weight of the fatty acid methyl ester composition, of C12-14 fatty acid methyl esters.

In a preferred embodiment, the fatty acid methyl ester composition comprises at least 2 wt.-% of C10 fatty acid methyl esters, at least 25 wt.-% of C12 fatty acid methyl esters, at least 5 wt.-% of C14 fatty acid methyl esters, and at least 4 wt.-% of C16 fatty acid methyl esters, each based on the total weight of the fatty acid methyl ester composition.

In a preferred embodiment, the fatty acid methyl ester composition comprises 0 to 5 wt.-% of C8 fatty acid methyl esters, 2 to 6 wt.-% of C10 fatty acid methyl esters, 25 to 45 wt.-% of C12 fatty acid methyl esters, 5 to 20 wt.-% of C14 fatty acid methyl esters, and 4 to 15 wt.-% of C16 fatty acid methyl esters, each based on the total weight of the fatty acid methyl ester composition.

In a preferred embodiment, the fatty acid methyl ester sulfonate composition comprises
1 to 20 of wt.-% of a C8 fatty acid methyl ester sulfonate,
1 to 8 of wt.-% of a C10 fatty acid methyl ester sulfonate,
30 to 48 wt.-% of a C12 fatty acid methyl ester sulfonate,
5 to 15 wt.-% of a C14 fatty acid methyl ester sulfonate,
4 to 13 wt.-% of a C16 fatty acid methyl ester sulfonate,
15 to 42 wt.-% of a C18 fatty acid methyl ester sulfonate, and
0 to 5 wt.-% of a C20 fatty acid methyl ester sulfonate,
each based on the total weight of the fatty acid methyl ester sulfonate composition. Said fatty acid methyl ester sulfonate composition is preferably obtained from oil extracted from Macaúba kernel.

In a preferred embodiment, the fatty acid methyl ester sulfonate composition comprises 3 to 7 wt.-%, preferably 4 to 6 wt.-%, of a C8 fatty acid methyl ester sulfonate,
2 to 6 wt.-%, preferably 3 to 5 wt.-%, of a C10 fatty acid methyl ester sulfonate,
36 to 46 wt.-%, preferably 38 to 42 wt.-%, of a C12 fatty acid methyl ester sulfonate,
6 to 13 wt.-%, preferably 8 to 11 wt.-%, of a C14 fatty acid methyl ester sulfonate,
5 to 11 wt.-%, preferably 6 to 9 wt.-%, of a C16 fatty acid methyl ester sulfonate,
25 to 40 wt.-%, preferably 30 to 38 wt.-% of a C18 fatty acid methyl ester sulfonate, and
0 to 4 wt.-%, preferably 0 to 3 wt.-%, of a C20 fatty acid methyl ester sulfonate,
each based on the total weight of the fatty acid methyl ester sulfonate composition. Said fatty acid methyl ester sulfonate composition is preferably obtained from oil extracted from Macaúba kernel.

In a preferred embodiment, the fatty acid methyl ester sulfonate composition comprises 0 to 5 wt.-%, preferably 0 to 3 wt.-%, and in particular 0 to 2 wt.-%, of a C10 fatty acid methyl ester sulfonate,
0 to 6 wt.-%, preferably 0 to 5 wt.-%, and in particular 1 to 4 wt.-%, of a C12 fatty acid methyl ester sulfonate,
0 to 6 wt.-%, preferably 0 to 5 wt.-%, and in particular 1 to 4 wt.-%, of a C14 fatty acid methyl ester sulfonate,
10 to 35 wt.-%, preferably 13 to 32 wt.-%, and in particular 15 to 30 wt.-%, of a C16 fatty acid methyl ester sulfonate,
55 to 85 wt.-%, preferably 60 to 80 wt.-%, and in particular 65 to 75 wt.-%, of a C18 fatty acid methyl ester sulfonate,
0 to 4 wt.-%, preferably 0 to 3 wt.-%, and in particular 0 to 2 wt.-%, of a C20 fatty acid methyl ester sulfonate,
each based on the total weight of the fatty acid methyl ester sulfonate composition. Said fatty acid methyl ester sulfonate composition is preferably obtained from oil extracted from Macaúba pulp.

In a preferred embodiment, the fatty acid methyl ester sulfonate composition comprises at least 85 wt.-% based on the total weight of the fatty acid methyl ester sulfonate composition, of C4-C22 fatty acid methyl ester sulfonates, preferably C10-C22 fatty acid methyl ester sulfonates, more preferably C12-C20 fatty acid methyl ester sulfonates, even more preferably C12-C20 fatty acid methyl ester sulfonates, and in particular C12-C18 fatty acid methyl ester sulfonates.

In a preferred embodiment, the fatty acid methyl ester sulfonate composition comprises at least 10 wt.-% of C16 fatty acid methyl ester sulfonates and at least 65 wt.-% of C18 fatty acid methyl ester sulfonates, each based on the total weight of the fatty acid methyl ester sulfonate composition.

In a preferred embodiment, the fatty acid methyl ester sulfonate composition comprises 10 to 40 wt.-% of C16 fatty acid methyl ester sulfonates and 60 to 90 wt.-% of C18 fatty acid methyl ester sulfonates, each based on the total weight of the fatty acid methyl ester sulfonate composition.

In a preferred embodiment, the fatty acid methyl ester sulfonate composition comprises at least 30 wt.-%, preferably at least 35 wt.-%, and in particular at least 40 wt.-%, based on the total weight of the fatty acid methyl ester sulfonate composition, of C12-14 fatty acid methyl ester sulfonates.

In a preferred embodiment, the fatty acid methyl ester sulfonate composition comprises at least 2 wt.-% of C10 fatty acid methyl ester sulfonates, at least 25 wt.-% of C12 fatty acid methyl ester sulfonates, at least 5 wt.-% of C14 fatty acid methyl ester sulfonates, and at least 4 wt.-% of C16 fatty acid methyl ester sulfonates, each based on the total weight of the fatty acid methyl ester sulfonate composition.

In a preferred embodiment, the fatty acid methyl ester sulfonate composition comprises 0 to 5 wt.-% of C8 fatty acid methyl ester sulfonates, 2 to 6 wt.-% of C10 fatty acid methyl ester sulfonates, 25 to 45 wt.-% of C12 fatty acid methyl ester sulfonates, 5 to 20 wt.-% of C14 fatty acid methyl ester sulfonates, and 4 to 15 wt.-% of C16 fatty acid methyl ester sulfonates, each based on the total weight of the fatty acid methyl ester sulfonate composition.

In a preferred embodiment, the fatty acid methyl ester sulfonates are selected from the group consisting of linear alkylbenzene sulfonates, alkyl sulfonates, and alpha olefin sulfonates.

Suitable linear alkylbenzene sulfonates can be expressed by the general formula (I)

CH3-(CH2)n-CHR1-(CH2)m-CO-O-CH3 (I),

wherein n is an integer from 0 to 21, m is an integer from 0 to 21, the sum of n and m is from 3 to 21, R1 is phenyl or benzyl, which is substituted with at least one R2, wherein R2 is SO3M, and wherein M is Na, K, or ammonium. In this connection, it is to be understood that SO3 comprises a negative charge and M comprises a positive charge.

Suitable alkyl sulfonates can be expressed by the general formula (II)

CH3-(CH2)i-CHR3-(CH2)j-CO-O-CH3 (II),

wherein i is an integer from 0 to 21, j is an integer from 0 to 21, the sum of i and j is from 3 to 21, R3 is C1-C10-alkyl, which is substituted with at least one R4, wherein R4 is SO3M, and wherein M is Na, K, or ammonium.

Suitable alpha olefin sulfonates can be expressed by the general formula (III)

R-CHSO3H-CO-O-CH3 (III),

wherein R is saturated or unsaturated C3-C23-alkyl, preferably C5-C19-alkyl.

In a preferred embodiment, the fatty acid methyl ester sulfonate is a beta olefin sulfonate, which can be expressed by the general formula (IV)

R-CHSO3H-CH2-CO-O-CH3 (IV),

wherein R is saturated or unsaturated C2-C22-alkyl, preferably C4-C18-alkyl.

As indicated above, the present invention further relates to a fatty acid methyl ester composition or a fatty acid methyl ester sulfonate composition obtained from the fruits of a plant having an oil yield in tons per hectare per year of at least 6 t/ha/yr, wherein the oil obtained from the plant is converted into the fatty acid methyl ester or its sulfonate.

Preferred embodiments (e.g. regarding the fatty acid methyl ester composition or the fatty acid methyl ester sulfonate composition and the plant) are already above-outlined in the process of manufacturing a fatty acid methyl ester composition or a fatty acid methyl ester sulfonate composition and in the further details regarding the fatty acid methyl ester composition or the fatty acid methyl ester sulfonate composition and shall apply for the fatty acid methyl ester composition or the fatty acid methyl ester sulfonate composition, as well. Particular preferred embodiment are mentioned in the following.

In a preferred embodiment, the plant is a palm, preferably a palm of the genus *Acrocomia,* more preferably a Macaúba palm, still more preferably *Acrocomia hassleri*, *Acrocomia totei,* and/or *Acrocomia aculeata,* and in particular *Acrocomia aculeata* and/or wherein the oil is obtained by extraction of the fruits, preferably wherein the plant is a palm and the oil is extracted from the palm pulp and/or the palm kernel, more preferably wherein the plant is Macaúba palm and the oil is extracted from the Macaúba kernel, still more preferably wherein the plant is *Acrocomia hassleri*, *Acrocomia totei,* and/or *Acrocomia aculeata* and the oil is extracted from more preferably *Acrocomia hassleri* kernel, *Acrocomia totei* kernel, and/or *Acrocomia aculeata* kernel, and in particular wherein the plant is *Acrocomia aculeata* and the oil is extracted from *Acrocomia aculeata* kernel.

In a preferred embodiment, the fatty acid methyl ester composition comprises
1 to 20 of wt.-% of a C8 fatty acid methyl ester,
1 to 8 of wt.-% of a C10 fatty acid methyl ester,
30 to 48 wt.-% of a C12 fatty acid methyl ester,
5 to 15 wt.-% of a C14 fatty acid methyl ester,
4 to 13 wt.-% of a C16 fatty acid methyl ester,
15 to 42 wt.-% of a C18 fatty acid methyl ester, and
0 to 5 wt.-% of a C20 fatty acid methyl ester,
each based on the total weight of the fatty acid methyl ester composition. Said fatty acid methyl ester composition is preferably obtained from oil extracted from Macaúba kernel.

In a preferred embodiment, the fatty acid methyl ester composition comprises
3 to 7 wt.-%, preferably 4 to 6 wt.-%, of a C8 fatty acid methyl ester,
2 to 6 wt.-%, preferably 3 to 5 wt.-%, of a C10 fatty acid methyl ester,
36 to 46 wt.-%, preferably 38 to 42 wt.-%, of a C12 fatty acid methyl ester,
6 to 13 wt.-%, preferably 8 to 11 wt.-%, of a C14 fatty acid methyl ester,
5 to 11 wt.-%, preferably 6 to 9 wt.-%, of a C16 fatty acid methyl ester,
25 to 40 wt.-%, preferably 30 to 38 wt.-% of a C18 fatty acid methyl ester, and
0 to 4 wt.-%, preferably 0 to 3 wt.-%, of a C20 fatty acid methyl ester,
each based on the total weight of the fatty acid methyl ester composition. Said fatty acid methyl ester composition is preferably obtained from oil extracted from Macaúba kernel.

In a preferred embodiment, the fatty acid methyl ester composition comprises
0 to 5 wt.-%, preferably 0 to 3 wt.-%, and in particular 0 to 2 wt.-%, of a C10 fatty acid methyl ester,
0 to 6 wt.-%, preferably 0 to 5 wt.-%, and in particular 1 to 4 wt.-%, of a C12 fatty acid methyl ester,
0 to 6 wt.-%, preferably 0 to 5 wt.-%, and in particular 1 to 4 wt.-%, of a C14 fatty acid methyl ester,
10 to 35 wt.-%, preferably 13 to 32 wt.-%, and in particular 15 to 30 wt.-%, of a C16 fatty acid methyl ester,
55 to 85 wt.-%, preferably 60 to 80 wt.-%, and in particular 65 to 75 wt.-%, of a C18 fatty acid methyl ester,
0 to 4 wt.-%, preferably 0 to 3 wt.-%, and in particular 0 to 2 wt.-%, of a C20 fatty acid methyl ester,
each based on the total weight of the fatty acid methyl ester composition. Said fatty acid methyl ester composition is preferably obtained from oil extracted from Macaúba pulp.

In a preferred embodiment, the fatty acid methyl ester composition comprises at least 85 wt.-% based on the total weight of the fatty acid methyl ester composition, of C4-C22 fatty acid methyl esters, preferably C10-C22 fatty acid methyl esters, more preferably C12-C20 fatty acid methyl esters, even more preferably C12-C20 fatty acid methyl esters, and in particular C12-C18 fatty acid methyl esters.

In a preferred embodiment, the fatty acid methyl ester composition comprises at least 10 wt.-% of C16 fatty acid methyl esters and at least 65 wt.-% of C18 fatty acid methyl esters, each based on the total weight of the fatty acid methyl ester composition.

In a preferred embodiment, the fatty acid methyl ester composition comprises 10 to 40 wt.-% of C16 fatty acid methyl esters and 60 to 90 wt.-% of C18 fatty acid methyl esters, each based on the total weight of the fatty acid methyl ester composition.

In a preferred embodiment, the fatty acid methyl ester composition comprises at least 30 wt.-%, preferably at least 35 wt.-%, and in particular at least 40 wt.-%, based on the total weight of the fatty acid methyl ester composition, of C12-14 fatty acid methyl esters.

In a preferred embodiment, the fatty acid methyl ester composition comprises at least 2 wt.-% of C10 fatty acid methyl esters, at least 25 wt.-% of C12 fatty acid methyl esters, at least 5 wt.-% of C14 fatty acid methyl esters, and at least 4 wt.-% of C16 fatty acid methyl esters, each based on the total weight of the fatty acid methyl ester composition.

In a preferred embodiment, the fatty acid methyl ester composition comprises 0 to 5 wt.-% of C8 fatty acid methyl esters, 2 to 6 wt.-% of C10 fatty acid methyl esters, 25 to 45 wt.-% of C12 fatty acid methyl esters, 5 to 20 wt.-% of C14 fatty acid methyl esters, and 4 to 15 wt.-% of C16 fatty acid methyl esters, each based on the total weight of the fatty acid methyl ester composition.

In a preferred embodiment, the fatty acid methyl ester sulfonate composition comprises
1 to 20 of wt.-% of a C8 fatty acid methyl ester sulfonate,
1 to 8 of wt.-% of a C10 fatty acid methyl ester sulfonate,
30 to 48 wt.-% of a C12 fatty acid methyl ester sulfonate,
5 to 15 wt.-% of a C14 fatty acid methyl ester sulfonate,
4 to 13 wt.-% of a C16 fatty acid methyl ester sulfonate,
15 to 42 wt.-% of a C18 fatty acid methyl ester sulfonate, and
0 to 5 wt.-% of a C20 fatty acid methyl ester sulfonate,
each based on the total weight of the fatty acid methyl ester sulfonate composition. Said fatty acid methyl ester sulfonate composition is preferably obtained from oil extracted from Macaúba kernel.

In a preferred embodiment, the fatty acid methyl ester sulfonate composition comprises 3 to 7 wt.-%, preferably 4 to 6 wt.-%, of a C8 fatty acid methyl ester sulfonate,
2 to 6 wt.-%, preferably 3 to 5 wt.-%, of a C10 fatty acid methyl ester sulfonate,
36 to 46 wt.-%, preferably 38 to 42 wt.-%, of a C12 fatty acid methyl ester sulfonate,
6 to 13 wt.-%, preferably 8 to 11 wt.-%, of a C14 fatty acid methyl ester sulfonate,
5 to 11 wt.-%, preferably 6 to 9 wt.-%, of a C16 fatty acid methyl ester sulfonate,
25 to 40 wt.-%, preferably 30 to 38 wt.-% of a C18 fatty acid methyl ester sulfonate, and
0 to 4 wt.-%, preferably 0 to 3 wt.-%, of a C20 fatty acid methyl ester sulfonate,
each based on the total weight of the fatty acid methyl ester sulfonate composition. Said fatty acid methyl ester sulfonate composition is preferably obtained from oil extracted from Macaúba kernel.

In a preferred embodiment, the fatty acid methyl ester sulfonate composition comprises
0 to 5 wt.-%, preferably 0 to 3 wt.-%, and in particular 0 to 2 wt.-%, of a C10 fatty acid methyl ester sulfonate,
0 to 6 wt.-%, preferably 0 to 5 wt.-%, and in particular 1 to 4 wt.-%, of a C12 fatty acid methyl ester sulfonate,
0 to 6 wt.-%, preferably 0 to 5 wt.-%, and in particular 1 to 4 wt.-%, of a C14 fatty acid methyl ester sulfonate,
10 to 35 wt.-%, preferably 13 to 32 wt.-%, and in particular 15 to 30 wt.-%, of a C16 fatty acid methyl ester sulfonate,
55 to 85 wt.-%, preferably 60 to 80 wt.-%, and in particular 65 to 75 wt.-%, of a C18 fatty acid methyl ester sulfonate,
0 to 4 wt.-%, preferably 0 to 3 wt.-%, and in particular 0 to 2 wt.-%, of a C20 fatty acid methyl ester sulfonate,
each based on the total weight of the fatty acid methyl ester sulfonate composition. Said fatty acid methyl ester sulfonate composition is preferably obtained from oil extracted from Macaúba pulp.

In a preferred embodiment, the fatty acid methyl ester sulfonate composition comprises at least 85 wt.-% based on the total weight of the fatty acid methyl ester sulfonate composition, of C4-C22 fatty acid methyl ester sulfonates, preferably C10-C22 fatty acid methyl ester sulfonates, more preferably C12-C20 fatty acid methyl ester sulfonates, even more preferably C12-C20 fatty acid methyl ester sulfonates, and in particular C12-C18 fatty acid methyl ester sulfonates.

In a preferred embodiment, the fatty acid methyl ester sulfonate composition comprises at least 10 wt.-% of C16 fatty acid methyl ester sulfonates and at least 65 wt.-% of C18 fatty acid methyl ester sulfonates, each based on the total weight of the fatty acid methyl ester sulfonate composition.

In a preferred embodiment, the fatty acid methyl ester sulfonate composition comprises 10 to 40 wt.-% of C16 fatty acid methyl ester sulfonates and 60 to 90 wt.-% of C18 fatty acid methyl ester sulfonates, each based on the total weight of the fatty acid methyl ester sulfonate composition.

In a preferred embodiment, the fatty acid methyl ester sulfonate composition comprises at least 30 wt.-%, preferably at least 35 wt.-%, and in particular at least 40 wt.-%, based on the total weight of the fatty acid methyl ester sulfonate composition, of C12-14 fatty acid methyl ester sulfonates.

In a preferred embodiment, the fatty acid methyl ester sulfonate composition comprises at least 2 wt.-% of C10 fatty acid methyl ester sulfonates, at least 25 wt.-% of C12 fatty acid methyl ester sulfonates, at least 5 wt.-% of C14 fatty acid methyl ester sulfonates, and at least 4 wt.-% of C16 fatty acid methyl ester sulfonates, each based on the total weight of the fatty acid methyl ester sulfonate composition.

In a preferred embodiment, the fatty acid methyl ester sulfonate composition comprises 0 to 5 wt.-% of C8 fatty acid methyl ester sulfonates, 2 to 6 wt.-% of C10 fatty acid methyl ester sulfonates, 25 to 45 wt.-% of C12 fatty acid methyl ester sulfonates, 5 to 20 wt.-% of C14 fatty acid methyl ester sulfonates, and 4 to 15 wt.-% of C16 fatty acid methyl ester sulfonates, each based on the total weight of the fatty acid methyl ester sulfonate composition.

As indicated above, the present invention further relates to the use of oil extracted from fruits of a plant having an oil yield in tons per hectare per year of at least 6 t/ha/yr for manufacturing a fatty acid methyl esters or sulfonates thereof.

Preferred embodiments (e.g. regarding the fatty acid methyl ester composition or the fatty acid methyl ester sulfonate composition and the plant) are already above-outlined in the process of manufacturing a fatty acid methyl ester composition or a fatty acid methyl ester sulfonate composition and in the further details regarding the fatty acid methyl ester composition or the fatty acid methyl ester sulfonate composition and shall apply for the use, as well.

As indicated above, the present invention further relates to the use of the above-outlined fatty acid methyl ester composition or the above-outlined fatty acid methyl ester sulfonate composition, in a personal care composition, a cleaning composition, a nutrition formulation, a pharmaceutical formulation, or a crops formulation. Preferably, the above-outlined fatty acid methyl ester composition or the above-outlined fatty acid methyl ester sulfonate composition are used as surfactant.

In a preferred embodiment, the above-outlined surfactants are used in a personal care composition, preferably selected from the group consisting of face care composition, hair care composition, body care composition, oral care composition, or antiperspirants and deodorants.

Suitable cosmetic formulations containing active ingredients are, e.g., hormone preparations, vitamin preparations, vegetable extract preparations and antibacterial preparations.

According to the present invention the personal care composition may comprise one or more active agent(s), e.g., organic and inorganic UV filters and vitamins, as well as other ingredients or additives, e.g., pigments, emulsifiers, emollients, viscosity regulators, stabilizers, preservatives, or fragrances.

In a preferred embodiment, the above-outlined surfactants are used in a sunscreen.

In a preferred embodiment, the above-outlined surfactants are used in a decorative preparations, preferably selected from the group consisting of lipsticks, nail varnishes, eye shadows, mascaras, dry and moist make-up, rouge, powders, depilatory agents and suntan lotions.

The personal care composition is preferably in form of creams, ointments, pastes, foams, gels, lotions, powders, make-ups, sprays, sticks or aerosols.

Preferably, the surfactant is used to control the metal ions, improve the dispersing, improve the emulsifying, control the foaming, modify the surface, and/or protect the active agent(s).

In a preferred embodiment, the above-outlined surfactants are used in a cleaning composition, preferably selected from the group consisting of home care formulation, industrial care formulation, and institutional care formulation.

In a preferred embodiment, the cleaning composition is selected from the group consisting of laundry composition (personal and commercial), dishwashing composition, hard surface cleaning composition, food service and kitchen hygiene composition, food and beverage processing composition, sanitation composition, institutional cleaning composition, industrial cleaning composition, and vehicle and transportation care composition.

The cleaning composition may comprise at least one bleaching system known in the art in an amount of from 0 to 50 wt.-%. Suitable bleaching components include bleaching catalysts, photobleaches, bleach activators, sources of hydrogen peroxide such as sodium percarbonate and sodium perborates, preformed peracids, and mixtures thereof.

The cleaning compositions may furthermore comprise dirt-suspending agents, for example sodium carboxymethylcellulose; pH regulators, for example alkali metal or alkaline earth metal silicates; bactericides; foam regulators, for example soap; salts for adjusting the spray drying and the granulating properties, for example sodium sulfate; fragrances; antistatic agents; fabric conditioners; further bleaching agents; pigments; and/or toning agents.

Preferably, the surfactant is used to control the metal ions, improve the dispersing, improve the emulsifying, control the foaming, modify the surface, and/or protect the ingredient(s).

In a preferred embodiment, the above-outlined surfactants are used in a nutrition formulation, preferably from the group selected from food formulations and feed formulations. The nutrition formulation can have any suitable form, e.g. liquid or solid and can be administered or uptaken in any suitable manner, e.g. orally, parenterally, or rectally.

For the preparation of a nutrition formulation, or a premix or a precursor, the process may comprise mixing a stabilized solid and/or liquid formulation comprising at least one or more food substance(s) and at least one additional ingredient(s) such as stabilizing agent.

Suitable stabilizing agents may be selected from the group consisting of gummi arabicum, at least one plant protein and mixtures thereof. It is understood that the stabilizing agent can be selected from one agent, e.g. only gummi arabicum or be composed of a mixture of e.g. one plant protein and gummi arabicum or a mixture of two or three or more different plant proteins. In one embodiment, the stabilizing agent is gummi arabicum. In another embodiment, the stabilizing agent is at least one plant protein.

Preferably, the surfactant is used to control the metal ions, improve the dispersing, improve the emulsifying, control the foaming, modify the surface, and/or protect the ingredient(s).

In a preferred embodiment, the above-outlined surfactants are used in pharmaceutical formulation. The pharmaceutical formulation may be administered in any suitable manner such as by oral, transdermal, parenteral, nasal, vaginal, or rectal application. Suitable solid pharmaceutical formulation can be in form of tablets, suppositories, or capsules or in form of a spray. Suitable transdermal pharmaceutical formulations encompass patches or formulations such as sprays, lotions, creams, oils, foams, ointments, powders, or gels. Suitable liquid pharmaceutical formulations are preferably administered orally, parenterally, or nasal.

The pharmaceutical formulation is preferably in solid, semi-solid, or liquid form, preferably in form of tablets, suppositories, capsules, patches, as sprays, lotions, creams, oils, foams, ointments, powders, gels, or fluid.

The pharmaceutical formulation comprises at least one active agent, e.g. selected from the group consisting of anti-cancer agent, hormone, antiviral agent, antifungal agent, antibacterial agent, and inhibitor.

Preferably, the surfactant is used to control the metal ions, improve the dispersing, improve the emulsifying, control the foaming, modify the surface, and/or protect the active agent(s).

In a preferred embodiment, the above-outlined surfactants are used in crop formulation, preferably selected from the group consisting of pesticide formulations, fungicide formulations, and herbicide formulations.

The crop formulation is preferably in solid, semi-solid, or liquid form. Preferably, the crop formulation is suitable for a ready to use spray.

In a preferred embodiment, the pesticide formulation comprises a pesticide selected from the group consisting of chlorpyrifos, endosulfan, imazalil, DDT, toxaphene, lindane, methoxychlor, dieldrin, kelthane, chlordane, Perthane, endrin, aldrin, and heptachlor.

In a preferred embodiment, the fungicide formulation comprises a fungicide selected from the group consisting of azoxystrobin, pyraclostrobin, fluoxastrobin, trifloxystrobin, picoxystrobin, epoxiconazole, prothioconazole, myclobutanil, tebuconazole, propiconazole, cyproconazole, fenbuconazole, boscalid, penthiopyrad, bixafen, isopyrazam, sedaxane, fluopyram, and thifluzamide.

In a preferred embodiment, the herbicide formulation comprises a herbicide selected from the group consisting of glyphosate, glufosinate, imidazolinone (such as imazamethabenz, imazamox, imazapic (e.g. Kifix), imazapyr, imazaquin and imazethapyr), and cyclohexanediones (such as tepraloxydim and clethodim).

Suitable herbicide formulation show enhanced herbicide action against undesirable harmful plants, in particular against *Acalypha* species such as *Acalypha indica*, *Dinebra* species such as *Dinebra Arabica, Cynotis spec such as Cynotis axillaris*, *Parthenium spec such as Parthenium hysterophorus, Physalis spec such as Physalis minima, Digera spec such as Digera arvensis, Alopecurus myosuroides*, *Apera spicaventi*, *Brachiaria* spec. such as *Brachiaria deflexa* or *Brachiaria plantaginea*, *Echinochloa* spec. such as *Echinochloa colonum, Leptochloa* spec. such as *Leptochloa fusca*, Rottboellia cochinchinensis, *Digitaria sanguinalis, Eleusine indica,* Saccharum spontaneum, Cynodon dactylon, *Euphorbia hirta, Euphorbia geniculata, Commelina benghaiensis, Commelina communis, certain undesired Oryza spec. such as weedy rice or red rice (Oryza sativa), Phaiaris spec. such as Phaiaris canariensis,* Celosia argentea, Xanthium strumarium, *Papaver rhoeas*, *Geranium spec*, *Brassica spec*, *Avenafatua*, *Bromus* spec*.*, *Lolium* spec., *Phalaris* spec., *Setaria* spec., *Digitaria* spec., *brachiaria* spec., *Amaranthus* spec., *Chenopodium* spec., *Abutilon theophrasti*, *Galium aparine*, *Veronica* spec., or *Solanum* spec. and/or to improve their compatibility with crop plants, such as soybean, peanut, pea, bean, lentil, green gram, black gram, cluster bean, fenugreek, palm, other pulse or leguminous crops, or crops which are tolerant to the action of acetohydroxyacid synthase inhibiting herbicides, such as for example Clearfield^{®} wheat, Clearfield^{®} barley, Clearfield^{®} corn, Clearfield^{®} lentil, Clearfield^{®} oilseed rape or canola, Clearfield^{®} rice, Cultivance^{®} soybean and/or Clearfield^{®} sunflower. The formulation should also have a good pre-emergence herbicidal activity.

Preferably, the surfactant is used to control the metal ions, improve the dispersing, improve the emulsifying, control the foaming, modify the surface, and/or protect the crop.

In a preferred embodiment, the personal care composition, the cleaning composition, the nutrition formulation, the pharmaceutical formulation, or the crop formulation comprises at least two surfactants. In this connection it is to be understood that the personal care composition, the cleaning composition, the nutrition formulation, the pharmaceutical formulation, or the crop formulation may comprise at least two above-outlined surfactant, at least three of the above-outlined surfactant or at least one of the above-outlined surfactant in combination with at least one further, different surfactant. The at least one further, different surfactant may be fatty alcohol-based surfactants or fatty acid-based surfactants such as sulfonates, amides, isethionates, taurates, glycolipids, amino acids, esterquats, sophorolipids, rhamnolipids, amphoacetates, betains, amido alkanolamides, and alkoxylated fatty acid ester.

Potential mixtures of one of the above-outlined surfactants are listed in the following.

| | | | |
|---|---|---|---|
| 1 | + fatty acid methyl ester or its sulfonate | 12 | + amphotenside |
| 2 | + alkanolamide | 13 | + sulfoacetate |
| 3 | + isethionate | 14 | + alkylbetaine |
| 4 | + N-acyl-aminoacid (e.g. N-acylglutamic acid) | 15 | + alkylethoxylate |
| 5 | + taurate | 16 | + cationic polymer |
| 6 | + aminooxide | 17 | + cationic surfactant |
| 7 | + sulfonate | 18 | + silicone |
| 8 | + carboxylate | 19 | + sulfonated fatty acid salts |
| 9 | + sulfosuccinate | 20 | + proteinhydrolysate |
| 10 | + (alkylether)sulfate | 21 | + protein-derivative |
| 11 | + betaine (e.g. cocamindopropylbetaine) | 22 | + fatty alkyl polyglucoside |

As indicated above, the invention further relates to a personal care composition, a cleaning composition, a nutrition formulation, a pharmaceutical formulation, or a crop formulation comprising the above-outlined fatty acid methyl ester composition or the above-outlined fatty acid methyl ester sulfonate composition. It is to be understood that the further specification of the use of the above-outlined fatty acid methyl ester composition or the above-outlined fatty acid methyl ester sulfonate composition in the respective personal care composition, a cleaning composition, a nutrition formulation, a pharmaceutical formulation, or a crop formulation also applies for the personal care composition, a cleaning composition, a nutrition formulation, a pharmaceutical formulation, or a crop formulation.

### Examples

The present invention is further illustrated by the following prophetic examples.

The following examples are considered for the Macaúba palm (e.g. having registration number AEB402A) having an oil yield in tons per hectare per year of about 9.0 t/ha/yr.

### Example 1

The Macaúba palm is planted on a cattle field, e.g. about 380 trees per hectare. No deforestation is needed since the Macaúba palms are cultivated on already existing fields (silvopastoral) and the farmer can in addition to cattle breeding and/or milk production distribute the Macaúba fruits.

### Example 2

The Macaúba palm is planted on soya plantation (having a growth height of about 20 to 80 cm and an oil yield in tons per hectare per year about 0.6 t/ha/yr), e.g. about 340 trees per hectare. Again, no deforestation is needed since the Macaúba palms are cultivated on an already existing plantation (agroforestry). As the Macaúba palm grows up to about 15 meters in height, the soya can be cultivated parallel. In this connection, it is also possible to cultivate at least one more additional different plant (having a growth height of about 1 to 7 m) such as sunflower (having an oil yield in tons per hectare per year of about 0.7 t/ha/yr) or beans parallel.

As can be seen from the above examples, deforestation can be significantly reduced by cultivating Macaúba palms. Further, the biodiversity can be increased. In addition, even if the Macaúba palm is not cultivated as a monoculture, the total oil yield can be comparable with an oil palm (having an oil yield in tons per hectare per year of about 3.8 t/ha/yr) monoculture since the oil yield as above-defined of the Macaúba palm is higher. Without being bound to any theory, using a plant having an improved oil yield, degraded areas and springs and watersheds can more easily recover. Further, the retention of moisture in the soil is improved.

## Claims

1. A process of manufacturing a fatty acid methyl ester composition or a fatty acid methyl ester sulfonate composition, the process comprising the steps
a) converting oil extracted from a plant having an oil yield in tons per hectare per year of at least 6 t/ha/yr into fatty acid methyl esters,
b) optionally converting the fatty acid methyl esters into fatty acid methyl ester sulfonates.

2. The process according to claim 1, wherein the plant is a palm, preferably a Macaúba palm, and in particular *Acrocomia aculeata* and/or
wherein the plant is a palm and the oil is extracted from the palm pulp and/or the palm kernel, preferably wherein the plant is Macaúba palm and the oil is extracted from the Macaúba kernel, and in particular wherein the plant is *Acrocomia aculeata* and the oil is extracted from *Acrocomia aculeata* kernel.

3. The process according to claim 1 or 2, wherein in step a) the conversion is conducted under chemical or enzymatic conditions, preferably under chemical conditions and/or
wherein step a) involves a transesterification.

4. The process according to any one of claims 1 to 3, wherein the fatty acid methyl ester composition provided in step a) comprises at least 40 wt.-%, based on the total weight of the fatty acid methyl ester composition, of C4-C22 fatty acid methyl esters, preferably C6-C20 fatty acid methyl esters, more preferably C8-C18 fatty acid methyl esters, even more preferably C8-C16 fatty acid methyl esters or C16-C18 fatty acid methyl esters, and in particular C10-C16 fatty acid methyl esters
and/or
1 to 20 of wt.-% of a C8 fatty acid methyl ester,
1 to 8 of wt.-% of a C10 fatty acid methyl ester,
30 to 48 wt.-% of a C12 fatty acid methyl ester,
5 to 15 wt.-% of a C14 fatty acid methyl ester,
4 to 13 wt.-% of a C16 fatty acid methyl ester,
15 to 42 wt.-% of a C18 fatty acid methyl ester, and
0 to 5 wt.-% of a C20 fatty acid methyl ester,
each based on the total weight of the fatty acid methyl ester composition.

5. The process according to any one of claims 1 to 4, wherein the plant has an oil yield in tons per hectare per year in the range of 6 to 30 t/ha/yr, preferably 7 to 20 t/ha/yr, more preferably of 8 to 15 t/ha/yr.

6. The process according to any one of claims 1 to 5, wherein step b) is conducted and the fatty acid methyl ester sulfonates are selected from the group consisting of linear alkylbenzene sulfonates, alkyl sulfonates, and alpha olefin sulfonates.

7. The process according to any one of claims 1 to 6, wherein step a) further comprises the step
a.i) blending the oil extracted from a plant having an oil yield in tons per hectare per year of at least 6 t/ha/yr with an oil extracted from a plant having an oil yield in tons per hectare per year of less than 6 t/ha/yr, preferably wherein the oil extracted from a plant having an oil yield in tons per hectare per year of less than 6 t/ha/yr is derived from coconut oil (CNO), palm oil (PO), and/or palm kernel oil (PKO) and/or
wherein the fatty acid methyl ester composition obtained in step a) is blended with a fatty acid methyl ester composition obtained from an oil extracted from a plant having an oil yield in tons per hectare per year of less than 6 t/ha/yr and a subsequent conversion into the respective fatty acid methyl ester composition, preferably wherein the oil extracted from a plant having an oil yield in tons per hectare per year of less than 6 t/ha/yr is derived from coconut oil (CNO), palm oil (PO), and/or palm kernel oil (PKO).

8. The process according to any one of claims 1 to 7, wherein step b) is conducted and the fatty acid methyl ester sulfonate composition obtained in step b) is blended with a fatty acid methyl ester sulfonate composition obtained from oil extracted from a plant having an oil yield in tons per hectare per year of less than 6 t/ha/yr and a subsequent conversion into the respective fatty acid methyl ester sulfonate composition, preferably wherein the oil extracted from a plant having an oil yield in tons per hectare per year of less than 6 t/ha/yr is derived from coconut oil (CNO), palm oil (PO), and/or palm kernel oil (PKO).

9. A fatty acid methyl ester composition or a fatty acid methyl ester sulfonate composition obtained by a process according to any one of claims 1 to 8.

10. A fatty acid methyl ester composition or a fatty acid methyl ester sulfonate composition obtained from the fruits of a plant having an oil yield in tons per hectare per year of at least 6 t/ha/yr, wherein the oil obtained from the plant is converted into the fatty acid methyl ester or its sulfonate.

11. The fatty acid methyl ester composition or a fatty acid methyl ester sulfonate composition according to claim 10, wherein the plant is a palm, preferably a Macaúba palm, and in particular *Acrocomia aculeata* and/or wherein the oil is obtained by extraction of the fruits, preferably wherein the plant is a palm and the oil is extracted from the palm pulp and/or the palm kernel, more preferably wherein the plant is Macaúba palm and the oil is extracted from the Macaúba kernel, and in particular wherein the plant is *Acrocomia aculeata* and the oil is extracted from *Acrocomia aculeata* kernel.

12. Use of oil extracted from fruits of a plant having an oil yield in tons per hectare per year of at least 6 t/ha/yr for manufacturing a fatty acid methyl esters or sulfonates thereof.

13. Use of the fatty acid methyl ester composition or the fatty acid methyl ester sulfonate composition according to any one of claims 9 to 11 in a personal care composition, a cleaning composition, a nutrition formulation, a pharmaceutical formulation, or a crops formulation.

14. A personal care composition, a cleaning composition, a nutrition formulation, a pharmaceutical formulation, or a crop formulation comprising the fatty acid methyl ester composition or the fatty acid methyl ester sulfonate composition according to any one of claims 9 to 11.
